# EUROPEAN PATENT SPECIFICATION

(11) **EP 3 247 413 B1**
(45) Date of publication and mention of the grant of the patent: **25.11.2020**
(21) Application number: 16703018.8
(22) Date of filing: 20.01.2016
(51) Int. Cl.: A61L 27/24, A61L 27/36, A61L 27/48, A61L 27/56

(54) **TISSUE MATRICES WITH CONTROLLED POROSITY OR MECHANICAL PROPERTIES**
GEWEBEMATRIZEN MIT KONTROLLIERTER POROSITÄT ODER MECHANISCHEN EIGENSCHAFTEN
MATRICES TISSULAIRES À POROSITÉ CONTRÔLÉE OU AYANT DES PROPRIÉTÉS MÉCANIQUES CONTRÔLÉES

(30) Priority: 21.01.2015 US 201562105971 P; 16.04.2015 US 201562148532 P
(43) Date of publication of application: 29.11.2017
(73) Proprietor: LifeCell Corporation, Madison, New Jersey 07940 (US)
(72) Inventor: JESSOP, Israel, Annandale, NJ 08801 (US); SHAH, Mrinal, Parsippany, NJ 07054 (US); LEAMY, Patrick, Flemington, NJ 08822 (US)
(74) Representative: Forresters IP LLP
(86) International application number: PCT/US2016/014009
(87) International publication number: WO 2016/118558

(56) References cited:
- EP-A1- 1 216 717
- WO-A2-2010/059783
- WO-A2-2011/019822
- WO-A2-2012/017415
- US-A1- 2011 238 167
- US-A1- 2014 220 095

## Description

The present disclosure relates to tissue products, and more particularly, to tissue matrices that have been modified or combined with other materials to provide a desired modulus (compressive or tensile) or other controlled mechanical properties while allowing cellular ingrowth and tissue regeneration. The disclosure also includes tissue treatment products including one or more areas of modified (e.g., increased) fiber density and/or modified mechanical properties, as well as methods for making such products.

Various tissue-derived products are used to regenerate, repair, or otherwise treat diseased or damaged tissues and organs. Such products can include tissue grafts and/or processed tissues (e.g., acellular tissue matrices from skin, intestine, or other tissues, with or without cell seeding). Such products generally have properties determined by the tissue source (i.e., tissue type and animal from which it originated) and the processing parameters used to produce the tissue products. Since tissue products are often used for surgical applications and/or tissue replacement or augmentation, the products should support tissue growth and regeneration, as desired for the selected implantation site.
In order to provide desired biological properties, however, tissue products, including extracellular tissue matrices, must have structural and functional properties, including suitable porosity and chemical compositions, to facilitate cellular ingrowth and tissue regeneration without excessive fibrosis or scarring. In addition, to serve as tissue fillers, tissue products must possess sufficient rigidity to prevent undesirable compression or deformation upon implantation, while allowing for a favorable feel or aesthetic appearance.

For certain applications, however, presently available tissue products, such as acellular tissue matrices or synthetic materials, may have less than optimal mechanical properties or may become altered in vivo (e.g., by becoming weakened due to protease activity) too rapidly after implantation. For example, certain tissue products may be compressed too easily to act as tissue fillers or may become weakened or possess insufficient tensile strengths needed for certain load-bearing applications.

In addition, existing extracellular matrix product designs may be less than optimal for treatment of some conditions. For example, existing designs may be either too stiff to use in treatment of soft tissue or are too soft or inelastic to retain the volume and pore size necessary for durable soft tissue regeneration. Furthermore, durable, regenerative soft tissue fillers must maintain their volume by resisting external compressive forces while simultaneously providing the appropriate cellular mechanical cues to repopulate the scaffold with soft tissues (such as adipose) instead of harder fibrous or mineralized tissues.

In addition, some existing extracellular matrix products may have various mechanical properties, including a feel, stretchability, or pliability, that may be improved for some uses. For example, some collagen-based materials or extracellular matrix products may exhibit an initial stretch in a so-called toe-region (see, US Patent Publication No. US20090306790 A1, to Sun et al., at Fig. 1). And for certain applications, this initial stretch may be undesirable because it may require additional effort to appropriate tension the material during implantation for load-bearing applications. WO 2010/059783 discloses an implantable medical device comprising a composite graft material.

The present disclosure provides improved tissue products that have a desired compressive or tensile moduli, include improved handling properties, and/or maintain the ability to support cellular ingrowth and tissue regeneration.

### SUMMARY

The invention is defined by the claims.

The present disclosure provides improved devices for treating tissues. The devices can include one or more (first) components selected to permit cellular ingrowth and tissue regeneration at various anatomic sites. In addition, the devices can include one or more additional (second) components or modified regions that include variations in one or more of density, chemical structure, strength, modulus of elasticity, and porosity. The first components and second components can be formed into a three-dimensional structure (e.g., as a composite material), that has controlled macroscopic mechanical properties (e.g., a controlled compressive modulus) that is selected for a desired application (e.g., as a tissue filler to replace tissue removed during lumpectomy or other surgery). In addition, while providing the desire mechanical properties over a relevant period after implantation, the device can allow cellular ingrowth and tissue regeneration.

According to the invention, a tissue product is provided. The tissue product includes a first component comprising a collagen-containing matrix capable of supporting cellular ingrowth and tissue regeneration when implanted in a patient. In addition, the tissue product includes a second component comprising a three-dimensional structure, wherein the first component is embedded in a lattice framework of the second component. The second component can have a compressive modulus that is greater than a compressive modulus of the first component. According to the invention, the first component is an acellular tissue matrix and the second component is a synthetic polymeric material substrate.

According to certain embodiments, a tissue product is provided. The product can include a first component comprising a collagen-containing extracellular tissue matrix capable of supporting cellular ingrowth and tissue regeneration when implanted in a patient. In addition, the product can comprise a group of higher-modulus regions spaced throughout the first component, wherein the higher-modulus regions are formed of the collagen-containing extracellular matrix and have been processed such that regions have higher densities of collagen fibers than other portions of the tissue product.

In addition, the present disclosure provides methods of producing tissue products. According to one embodiment, the method includes selecting a first biocompatible material comprising a collagen-based extracellular matrix material having a porosity and microstructure selected to permit cellular ingrowth when implanted in a mammalian soft tissue. In addition, the method can include selecting a second biocompatible material having a density and a compressive modulus that is greater than a density and compressive modulus of the first biocompatible material. The method can further include forming a composite material from the first biocompatible material and the second biocompatible material.

In some embodiments the methods of producing the tissue product can comprise selecting a collagen-containing extracellular tissue matrix capable of supporting cellular ingrowth and tissue regeneration when implanted in a patient, and processing the collagen-containing extracellular tissue matrix to produce a group of regions spaced throughout the tissue product having a higher-modulus and/or higher density of collagen fibers.

### DESCRIPTION OF THE DRAWINGS

Fig. 1A is a perspective view of a hierarchically porous tissue matrix produced according to various exemplary embodiments.
Fig. 1B is an enlarged view of the tissue matrix of Fig. 1A.
Fig. 1C is a perspective view of a hierarchically porous tissue matrix produced according to various exemplary embodiments.
Fig. 2 is a perspective view of another hierarchically porous tissue matrix produced according to various exemplary embodiments.
Fig. 3A is a side view of a tissue matrix and process for forming a heirachically porous or compression-resistant tissue matrix.
Fig. 3B is a side view of a heirachically porous or compression-resistant tissue matrix produced according to the method illustrated in Fig. 3A.
Fig. 4A is a side elevated view of a tissue matrix having variations in porosity and mechanical properties (e.g., tensile or compressive properties) produced according to various embodiments.
Fig. 4B is a perspective view of another tissue matrix having variations in porosity and mechanical properties (e.g., tensile or compressive properties) produced according to various embodiments.
Fig. 5A is a hematoxylin and eosin (H&E) stained section of processed adipose tissue prior to implantation, as describe in the Example below.
Fig. 5B is an H&E stained section of a processed adipose tissue/polyurethane composite material prior to implantation, as describe in the Example below.
Figs. 6A-6B are H&E stained sections of processed adipose tissue after explantation, as describe in the Example below.
Figs 7A-7B are H&E stained sections of a polyurethane material after explantation, as describe in the Example below.
Figs 8A-8B are H&E stained sections of processed adipose tissue/polyurethane composite material after explantation, as describe in the Example below.
Fig. 9A is a side view of a tissue matrix and process for forming a tissue matrix having variation in collagen density, mechanical properties, and/or crosslinking.
Fig. 9B is a side view of a tissue matrix and process for forming a tissue matrix having variations in collagen density, mechanical properties, and/or crosslinking.
Fig. 9C is a side view of a tissue produce produced according to the method illustrated in Figs. 9A-9B.
Fig. 10A is a side view of a tissue matrix and process for forming a tissue matrix having variations in collagen density, mechanical properties, and/or crosslinking.
Fig. 10B is a side view of a tissue matrix produced using the matrix of Fig. 10A.
Fig. 11A illustrates a step in a method for producing a tissue matrix having variations in collagen density, mechanical properties, and/or crosslinking.
Fig. 11B illustrates a step in a method for producing a tissue matrix having variations in collagen density, mechanical properties, and/or crosslinking.
Fig. 11C illustrates a step in a method for producing a tissue matrix having variations in collagen density, mechanical properties, and/or crosslinking.
Fig. 11D illustrates a step in a method for producing a tissue matrix having variations in collagen density, mechanical properties, and/or crosslinking.

### DESCRIPTION OF CERTAIN EXEMPLARY EMBODIMENTS

Reference will now be made in detail to certain exemplary embodiments according to the present disclosure, certain examples of which are illustrated in the accompanying drawings. Wherever possible, the same reference numbers will be used throughout the drawings to refer to the same or like parts.

In this application, the use of the singular includes the plural unless specifically stated otherwise. In this application, the use of "or" means "and/or" unless stated otherwise. Furthermore, the use of the term "including", as well as other forms, such as "includes" and "included", is not limiting. Any range described herein will be understood to include the endpoints and all values between the endpoints.

The section headings used herein are for organizational purposes only and are not to be construed as limiting the subject matter described.

Various human and animal tissues can be used to produce products for treating patients. For example, various tissue products for regeneration, repair, augmentation, reinforcement, and/or treatment of human tissues that have been damaged or lost due to various diseases and/or structural damage (e.g., from trauma, surgery, atrophy, and/or long-term wear and degeneration) have been produced. Such products can include, for example, acellular tissue matrices, tissue allografts or xenografts, and/or reconstituted tissues (i.e., at least partially decellularized tissues that have been seeded with cells to produce viable materials).

A variety of tissue products have been produced for treating soft and hard tissues. For example, ALLODERM® and STRATTICE™ (LIFECELL CORPORATION, Branchburg, NJ) are two dermal acellular tissue matrices made from human and porcine dermis, respectively. Although such materials are very useful for treating certain types of conditions, materials having different biological and mechanical properties may be desirable for certain applications. For example, ALLODERM® and STRATTICE™ have been used to assist in treatment of structural defects and/or to provide support to tissues (e.g., for abdominal walls or in breast reconstruction), and their strength and biological properties make them well-suited for such uses.

Such materials, however, may not be ideal for regeneration, repair, replacement, and/or augmentation of certain soft-tissue defects. For example, improved tissue fillers for replacing lost or damaged tissues, including adipose or other soft tissues, may be beneficial for some patients. In addition, in some applications, tissue fillers may be improved by altering certain mechanical properties while maintaining the ability of the materials to support cellular in-growth and regeneration. Accordingly, improved devices and methods are provided, which allow alteration in one or more tissue scaffold mechanical and/or biological properties, while maintaining the ability to allow cellular in-growth and regeneration.

In certain embodiments, the devices include soft tissue fillers that resist external compression and pore collapse while maintaining extracellular matrix (ECM)-like mechanics on the cellular level. The devices can include mechanical-gradient structures as well as hierarchically porous structures in which regions with a relatively high compressive modulus are periodically dispersed into an otherwise soft extracellular matrix, resulting in a macro-mechanical modulus that is higher than the modulus of one or more components of the material.

According to various embodiments, a tissue product is provided. The tissue product can include a first component comprising a collagen-containing matrix capable of supporting cellular ingrowth and tissue regeneration when implanted in a patient. In addition, the tissue product can include a second component comprising a three-dimensional structure, wherein the first component is embedded in a lattice framework of the second component, and wherein the second component has a compressive modulus that is greater than a compressive modulus of the first component.

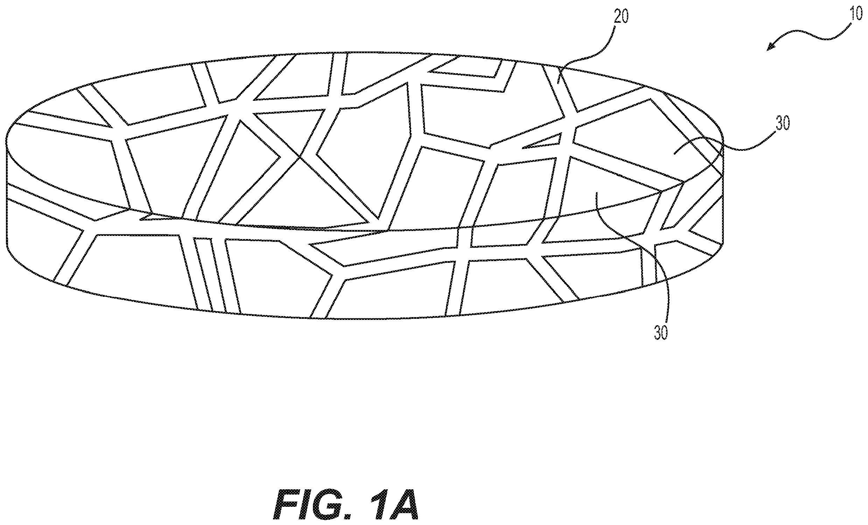

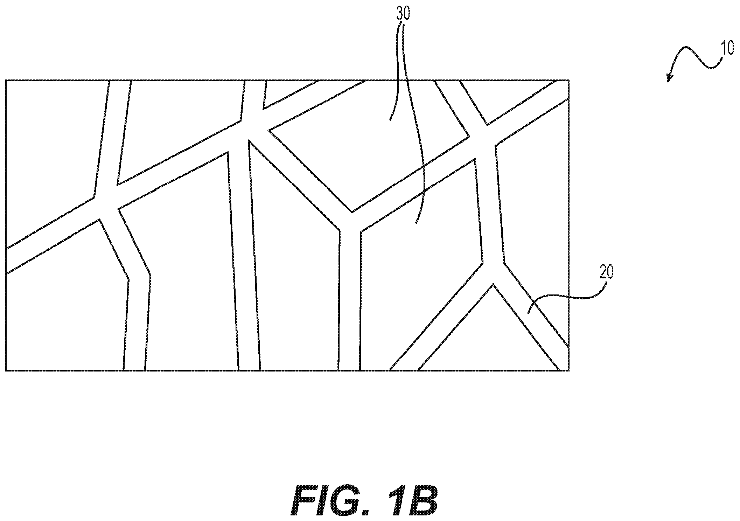

Fig. 1A is a perspective view of a hierarchically porous tissue matrix 10 produced according to various exemplary embodiments. Fig. 1B is an enlarged view of the tissue matrix of Fig. 1A. As shown, the tissue matrix 10 includes a first component 30 and a second component 20. The second component can comprise a three-dimensional structure, e.g., a lattice framework, and the first component can be embedded within the three-dimensional structure of the second component 20.

The first component 30 and the second component 20 can have differences in various structural, mechanical, and/or biological properties. For example, in certain embodiments, the first component 30 can include a porous material capable of allowing cellular ingrowth and tissue regeneration. The first component 30, however, may also be easily compressed as compared to the second component 20.

In order to control the macro-mechanical properties of the tissue matrix 10, the second component 20 may be less easily compressed than the first component 30. That is, the second component may have a higher compressive modulus, and may have variations in other mechanical, structural, or biological properties. For example, in certain embodiments, the second component 20 is formed of a material that is more dense than the first component 30, thereby resulting in a material that is less easily compressed, and which may also degrade or become resorbed more slowly in vivo.

The first component 30 and second component 20 can be formed from a variety of suitable materials. For example, in general, either component can be formed from bioabsorable synthetic materials such as polyurethane, polycaprolactone, or polylactic acid. Alternatively, either component may be formed of other polymers, including, but not limited to, polyurethanes, degradable polyesters, polyglycolic acid, polylactic acid, polycaprolactone, polytrimethylene carbonate and copolymers, polyanhydrides, polycarbonates, polyesteramides, polyphosphazenes, polyolefins, nondegradable polyesters, nylon, polyacrylates, silicones, tyrosine base polymers, polyhydroxyalkanoates, chitin, chitosan. In addition, either component may be formed from a variety of different collagen-based materials, including for example, extracellular matrix materials, micronized extracellular matrix, purified collagen, micronized collagen, defibrillated collagen, coarse collagen bundles, and collagen treated to control cross-linking (e.g., via chemical, thermal, photo, or radiation-induced cross-linking (as used herein, photo-or radiation-induced cross-linking can both refer to electromagnetic radiation in the visible range, and may also include cross-linking used non-visible radiation such as gamma, e-beam, or ultraviolet means). Various combinations of suitable materials will be described in more detail below.

As noted above, the first component 30 and/or second component 20 can be formed from a variety of different materials, including extracellular matrix products. For example, one or both of the components 20, 30 can be formed from acellular tissue matrices derived from a variety of different human or animal tissues. Suitable acellular matrix materials can include, for example, adipose tissue, dermal tissue, connective tissue (tendon, ligament, fascia), muscle (smooth or striated), bladder, liver, kidney, pancreas, neural tissue, vascular tissue (arterial or venous), or other soft tissue extracellular matrix, as well as demineralized bone matrix, or mineralized cancellous bone.

In addition, if extracellular matrix materials are used, the materials can be further processed to control desired properties, including porosity, mechanical properties (strength, compressibility, elasticity), and/or biological properties (e.g., enzyme susceptibility, in vivo degradation rate, and/or ability to support in growth and tissue regeneration for intended treatment sites). Such processing can include micronizing, re-suspension, cross-linking, defatting, decellularizing, and/or lyophilizing. Exemplary processing techniques for adipose and dermal materials are described further below.

Examples of various combinations of materials are described below. It will be understood that some of the materials listed can be interchanged in the various embodiments without departing from the intended scope of the invention.

In one embodiment, the second component 20 includes an open-celled foam material comprising a synthetic polymer such as polyurethanes, degradable polyesters, polyglycolic acid, polylactic acid, polycaprolactone, polytrimethylene carbonate and copolymers, polyanhydrides, polycarbonates, polyesteramides, polyphosphazenes, polyolefins, nondegradable polyesters, nylon, polyacrylates, silicones, tyrosine base polymers, polyhydroxyalkanoates, chitin, chitosan. The pores of the foam can have a range of suitable sizes, e.g., between 500 micrometers and 5000 micrometers, and the specific composition can be selected based on the desired mechanical properties. Furthermore, the specific pore size may be selected to allow ingrowth of cells from tissue to be treated. For example, pores of 1 to 20 microns may be used for nerve axons, and pores of 1-300 microns may be used for adipocytes.

The first component 30, which is to be embedded within the second component 20, can comprise a more easily compressed material. For example, the first component 30 can comprise a freeze-dried suspension of micronized extracellular matrix (e.g., adipose matrix, dermal matrix) within the polymer pores, non-crosslinked collagen, micronized collagen, non-mineralized collagen, and/or collagen-gag mixtures.

In another embodiment, the second component 20 can comprise a cross-linked collagen, and the first component 30 can comprise a non-crosslinked, or lightly cross-linked collagen, and freeze-dried suspension of micronized extracellular matrix (e.g., adipose matrix, dermal matrix) within the polymer pores, non-crosslinked collagen, micronized collagen, non-mineralized collagen, and/or collagen-gag mixtures. As such, the second component 20 will tend to be less compressible.

In another embodiment, the second component 20 comprises coarse collagen bundles, and the first component 30 can comprise a freeze-dried suspension of micronized extracellular matrix (e.g., adipose matrix, dermal matrix), non-crosslinked collagen, micronized collagen, non-mineralized collagen, and/or collagen-gag mixtures.

In another embodiment, the second component 20 comprises natural fibers such as silk or celluloses, and the first component 30 can comprise a freeze-dried suspension of micronized extracellular matrix (e.g., adipose matrix, dermal matrix), non-crosslinked collagen, lightly cross-linked collagen, micronized collagen, non-mineralized collagen, and/or collagen-gag mixtures.

In another embodiment, the second component 20 comprises mineralized collagen, and the first component 30 can comprise a freeze-dried suspension of micronized extracellular matrix (e.g., adipose matrix, dermal matrix), non-crosslinked collagen, lightly cross-linked collagen, micronized collagen, non-mineralized collagen, and/or collagen-gag mixtures.

Furthermore, the second component 20 or first component 30 can be formed from combinations of the aforementioned materials. In addition, either or both of the first component 30 and second component 20 may be processed subsequent to or before formation of the tissue matrix 10 to alter mechanical, structural, and/or biological properties. For example, in one embodiment, the second component 20 can be compressed and/or cross-linked to increase its density.

In some embodiments, the first component is embedded within portions s of the second component, but yet covers most or all of the surface of the second component. For example, the second component can include a synthetic polymeric material substrate, and the first component can include an acellular tissue matrix.

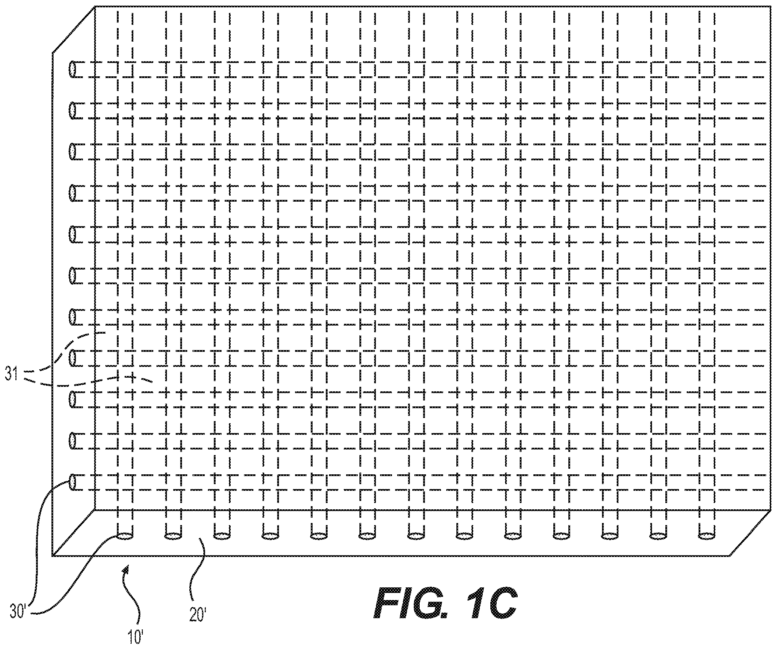

Fig. 1C illustrates such an embodiment. As shown the device of Fig. 1C includes a synthetic polymeric material substrate 30' and a tissue matrix coating or embedding material 20' while filling openings 31 in the substrate 30'. The material 20' can include any of the aforementioned tissue matrices and the substrate can similarly include any of the mentioned synthetics. In one embodiment the substrate 30' includes a polypropylene mesh, and the material 20' includes an adipose tissue matrix.

The substrate 30' can be formed from a specially designed mesh structure, or currently available mesh materials. Alternatively, the substrate can be formed from elongated elements such as surgical sutures (e.g., polypropylene sutures or bioresorbable sutures.

The embedding material can be formed in a number of ways. In one embodiment, the material 20' is formed by selecting an adipose tissue; treating the tissue to remove substantially all cellular material from the tissue; suspending the tissue in a liquid to form a suspension; placing the synthetic polymeric material substrate in the suspension; and freezing and drying the suspension to form a porous sponge embedding the synthetic polymeric material substrate.

After forming the material 20', the material 20' can be stabilized, e.g., by crosslinking (e.g., using chemical, UV, e-beam, gamma, x-ray, or other cross-linking processes) or by subjecting the material to a dehydrothermal process.

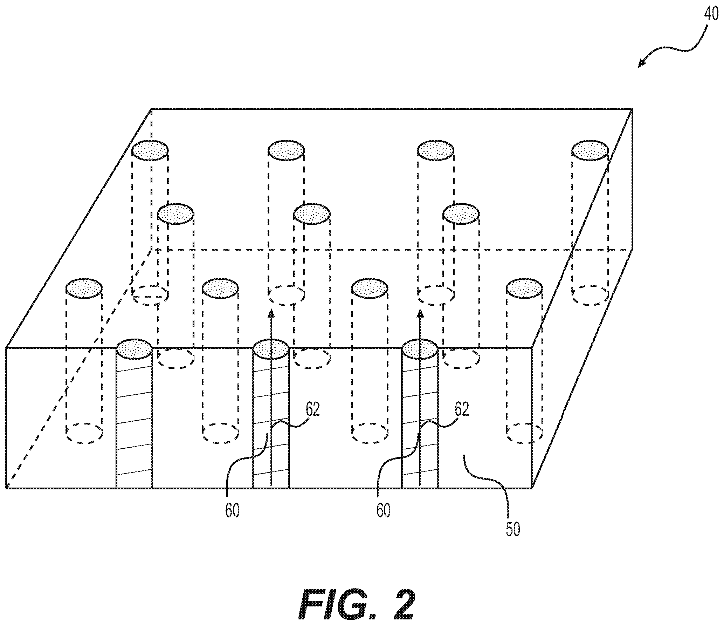

As noted above, in other embodiments, the devices disclosed herein can include regions of higher density and or higher compressive modulus. For example, Fig. 2 is a perspective view of another hierarchically porous tissue matrix 40 produced according to various exemplary embodiments. As shown, the tissue matrix 40, includes a first component 50 comprising a collagen-containing extracellular tissue matrix capable of supporting cellular ingrowth and tissue regeneration when implanted in a patient; and a group of higher-modulus regions 60 spaced throughout the first component 50, wherein the higher-modulus regions 60 are formed of the collagen-containing extracellular matrix and have been processed to produce regions of higher density.

The tissue matrix 40 and higher modulus regions 60 can have a variety of configurations. For example, as shown, the tissue matrix 40 comprises a flexible sheet-like material. It will be understood, however, that the tissue matrix can include a variety of shapes, including box shapes, cubic, pyramidal, ovoid, or irregular shapes selected based on a desired implantation site.

Furthermore, the higher modulus regions 60 can have a variety of shapes or configurations within the tissue matrix 40. For example, In some embodiments, most or all higher modulus regions 60 are aligned along a common axis 62, thereby imparting resistance to compression along a common direction.

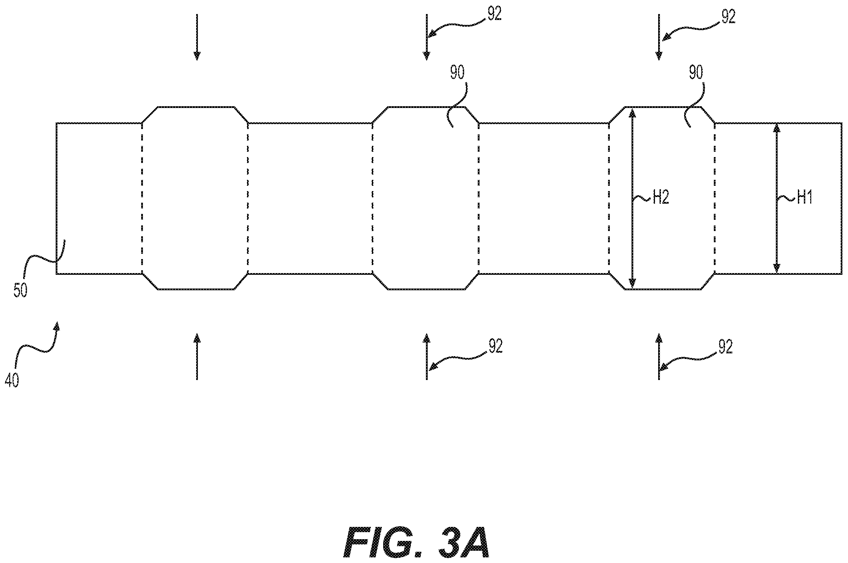

Figs. 3A-3B illustrate methods of producing the tissue matrix 40 of Fig. 2. As shown, in Fig. 3A, the tissue matrix 40 can be formed using a sheet-like piece of material have first regions 50 and second regions 90. The first regions 50 and second regions 90 can be formed of the same material (e.g., both formed from a sheet of acellular dermis, adipose tissue, or other suitable material such as collagen, small intestine, or muscle matrix), but can have different thicknesses H1 and H2.

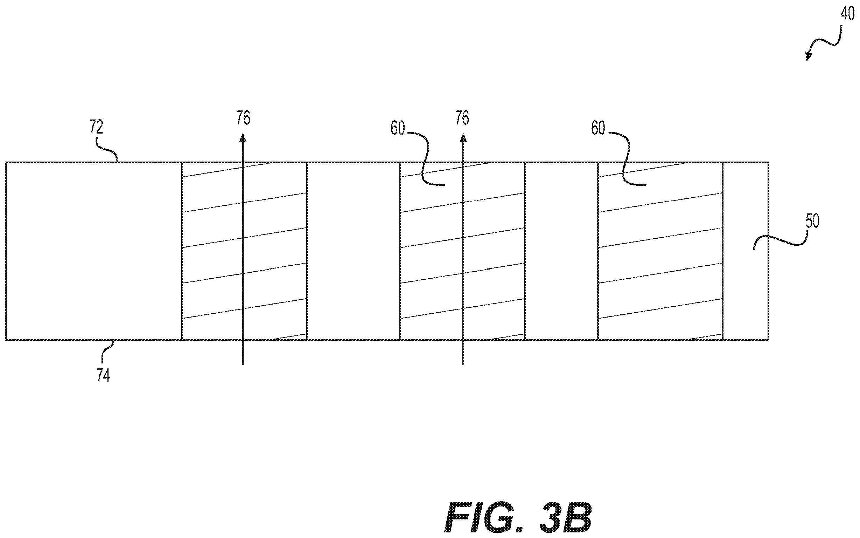

In order to produce regions of higher density, the second regions, which have a greater thickness H2, can be compressed by applying compressive forces 92 on opposing sides of the material. Subsequently, the compressed regions can be cross-linked, e.g., by radiation, chemical, thermal, or UV cross-linking, to stabilize the regions 90 in a compressed and more dense configuration. As such, the tissue matrix is formed into a final tissue matrix 40 having first regions 50, and second higher density regions 60, as shown in Fig. 3B. The second regions 60 can be formed to have at least one or both of a higher compressive modulus and a higher tensile modulus, as compared to the first regions 50. As such, the second regions can impart increased resistance to compression (e.g., to maintain tissue volume after implantation as a tissue filler) and/or to prevent tearing (e.g., when used under tensile loads for tissue regeneration).

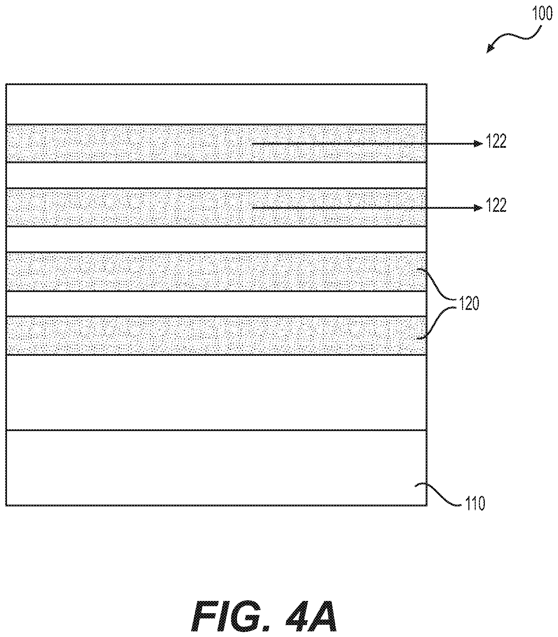

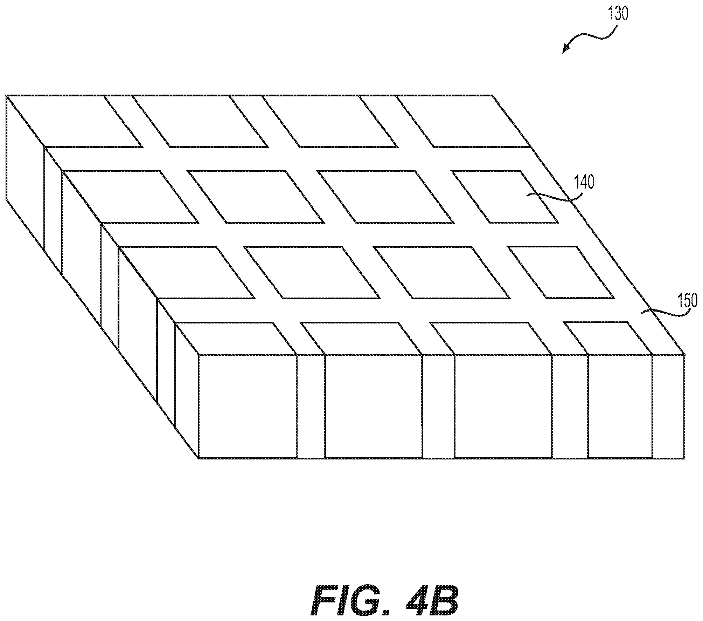

As shown, the tissue matrix 40 has a top surface 72 and 76, and the second regions 60 are formed as columns along an axis 76 perpendicular to the top and bottom surfaces. However, other configurations may be used. For example, Fig. 4A is a side elevated view of a tissue matrix 100 having variations in porosity and mechanical properties (e.g., tensile strength) produced according to various embodiments, and Fig. 4B is a perspective view of another tissue matrix 130 having variations in porosity and mechanical properties (e.g., tensile strength) produced according to various embodiments. In the embodiment of Fig. 4A, the device includes first regions 110 and second regions 120. The second regions 120 can include higher density areas, produced as discussed with reference to Fig. 3A, but can form elongated sections along direction 122. Similarly, the device of Fig. 4B can include first regions 140 and second regions 150, wherein the second regions are processed according to the method described with respect to Fig. 3A, to produce higher density regions in a grid-like shape. Other shapes and configurations may be selected based on the intended use.

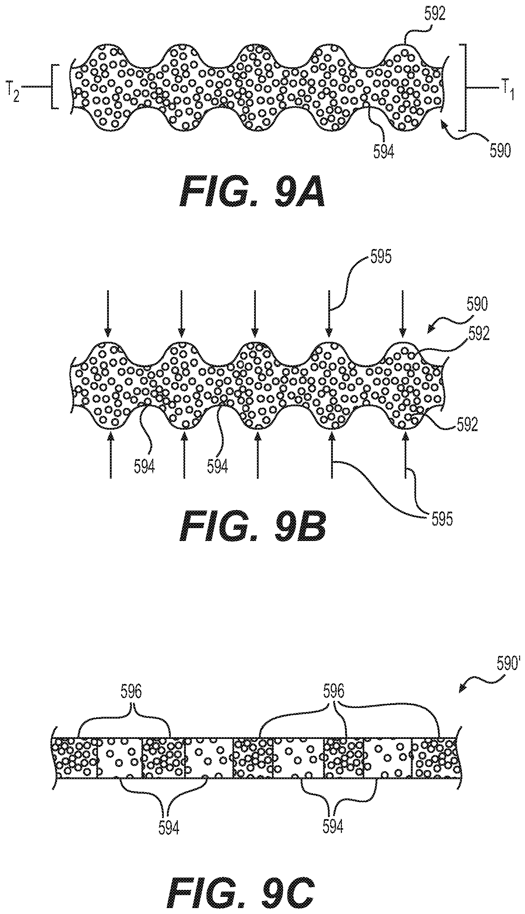

In certain embodiments, the present disclosure provides devices that can provide regions of increased collagen density and/or compression resistance after implantation in the body. For example, Fig. 9A-B illustrates a side view of a tissue matrix and process for forming a tissue matrix having variation in collagen density, mechanical properties, and/or crosslinking; and Fig. 9C is a side view of a tissue produce produced according to the method illustrated in Figs. 9A-9B.

As shown, Fig. 9A includes a contoured tissue matrix or other scaffold material 590 having first regions 592 of T1 and second regions of T2, wherein T2 is less than T1. Upon implantation, the first regions T1 will tend to experience compressive forces 595 from surrounding tissues, thereby shielding the first regions 594 from compression. Upon sufficient compression, the first regions 592 will produce regions 596 of higher density collagen.

It will be appreciated that the first 592 and second 594 regions can be formed of the same material, and production of the device without cross-linking or other material modification (other than forming the appropriate shape and thickness), but suitable modifications can be incorporated to further tailor the mechanical properties.

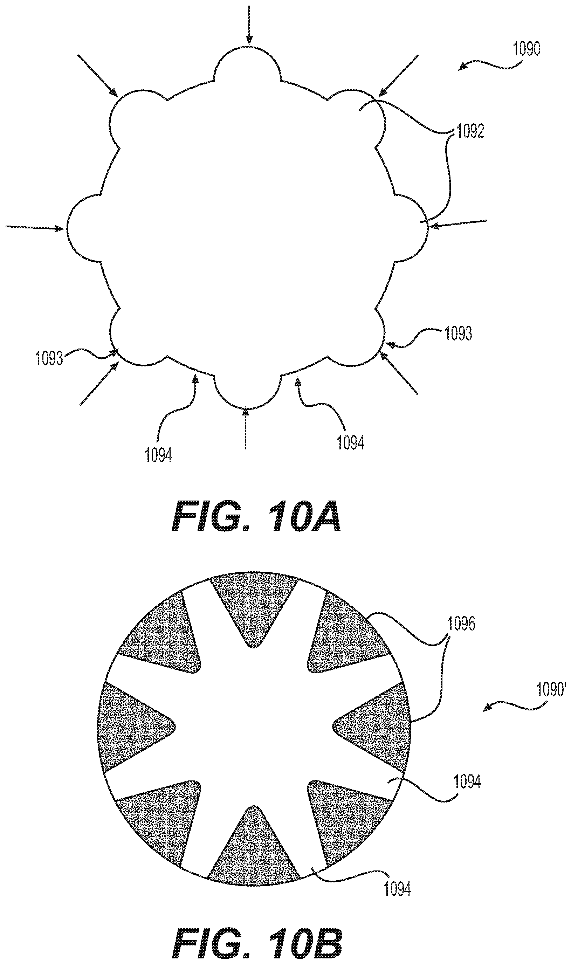

As noted above, the device disclosed herein, and illustrated with respect to Figs. 2-4B and 9A-C can include a variety of shapes. For example, Fig. 10A is a side view of a tissue matrix and process for forming a tissue matrix having variations in collagen density, mechanical properties, and/or crosslinking. Fig. 10B is a side view of a tissue matrix produced using the matrix of Fig. 10A.

The devices 1090 and 1090' can include a round or spherical shape (or similar 3-D structure), that can be configured to implant in various sites. In addition, the device can include first regions 1092 and second regions 1094, wherein the first regions have larger volumes or areas that can be compressed upon application of forces 1093.

Furthermore, the device 1090 can be compressed by application of forces 1093 and cross-linked, as discussed with respect to the devices of Fig. 2-4B to produce a final device 1090' having cross-linked section 1096 that are more dense and compression resistant. Alternatively, the device 1090 can be implanted with the regions 1092 protruding, and like the device of Fig. 10C, can be compressed upon implantation.

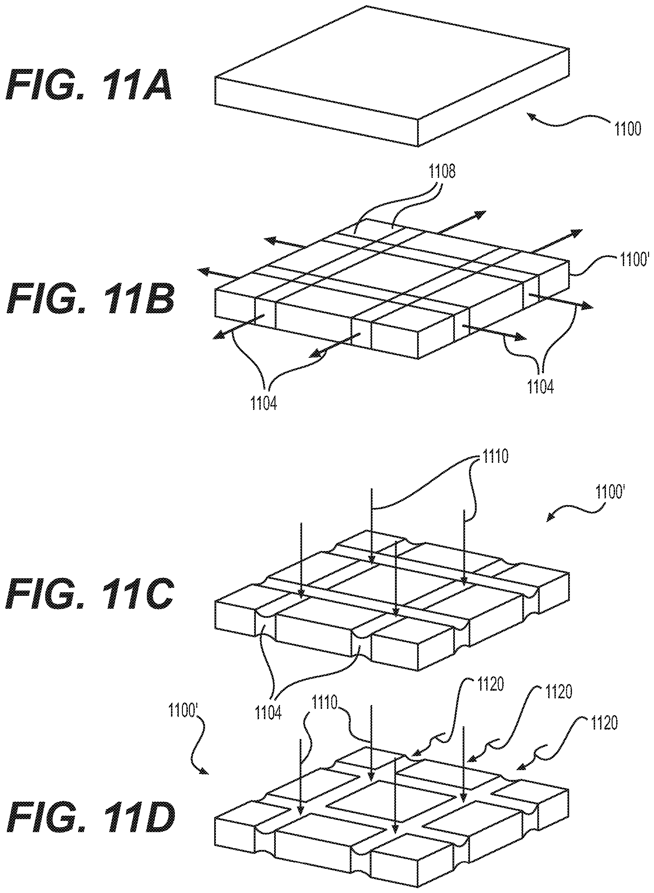

In addition to providing compressive forces for producing regions of higher density, tensile forces may be used in conjunction with cross-linking. For example, Figs. 11A-D illustrate additional methods for producing tissue matrices having variations in collagen density, mechanical properties, and/or crosslinking.

As shown, to produce a device with regions of higher density, a tissue matrix or other scaffold 1100 can be selected (Fig. 11A). To increase collagen fiber density in selected regions 1108, tensile forces 1104 can be applied to produce an intermediate material 1100' (Fig. 11B).

Subsequently, compressive forces 1110 can be applied (Fig. 11C) before release of the tensile forces, thereby maintaining the higher density regions, and crosslinking can be performed (Fig. 11D) by application of radiation 1120 or through another suitable process.

It should be noted that the compressive forces 1110 can be applied while the material is in a sterile shield that forms part of the final product packaging. As such, the material can be cross-linked without risking contamination.

It will also be appreciated that although the device of Figs. 2-3B are illustrated with higher density regions spaced within a matrix of lower density regions, the opposite can be true (i.e., lower density regions can be formed within a matrix of higher density regions). In addition, variations on the shapes and spacing of the various regions can be produced.

Furthermore, although the method of Figs. 3A-3B illustrates regions 90 as having thicknesses H2 greater than the thickness H1 of regions 50, this does not have to be the case. For example, a device 40 can be used with a relatively constant thickness such that the final device will have higher density regions 60 that are thinner after compression to produce a higher density.

### Methods of producing tissue products:

Methods of producing tissue products have been described with respect to the materials illustrated in Figs. 2-4B. The following description provides additional details regarding suitable methods for producing the products illustrated in Figs. 1A-1B. In one embodiment the method includes selecting a first biocompatible material comprising a collagen-based extracellular matrix material having a porosity and microstructure selected to permit cellular ingrowth when implanted in a mammalian soft tissue; selecting a second biocompatible material having a density and a compressive modulus that is greater than a density and compressive modulus of the first biocompatible material; and forming a composite material from the first biocompatible material and the second biocompatible material.

In one method, the second material can be formed into a pre-formed lattice, e.g. a lattice of synthetic polyurethane or other acceptable material, which may optionally be treated by cross-linking or other processes to control its properties. Subsequently, the first material can then be formed directly into the pre-formed lattice, e.g., by freeze-drying or stabilizing a flowable composition within the preformed lattice.

In a second method, the second material can be provided as a liquid suspension, and the first material can be provided as beads or particles. The first material can be placed into the liquid suspension, and the mixture can be freeze dried to produce a material having pieces of the first material embedded in a lattice of the second material.

In a third method, the first material can be provided as a liquid suspension, and the second material can be provided as beads or particles. The second material can be placed into the liquid suspension and the mixture can be freeze dried to produce a material having pieces of the second material embedded in a lattice of the first material.

In a fourth method, the second material can be provided as a frozen suspension of beads or rod, which can be coated with fibers of the first material. The composition can be compressing into the desired shape, and the compressed mixture can be freeze-dried.

In a fifth method, the first material can be provided as a preformed foam or tissue matrix. The second material can be provided as fibers struts or coils that can be mechanically embedded in the second material.

Finally, in a sixth method, the first material and second material can be provided as frozen beads, and the materials can be mixed in a controlled or random stacking pattern and freeze-dried to form the final material.

The materials described herein can be dried using conventional freeze-drying processes, and/or dehydrothermal processes to provide a small degree of cross-linking. Additionally or alternatively, the materials may be cross-linked using light, radiation, and/or chemical cross-linking.

### Alternative Embodiments and Methods of Producing Tissue Products:

### Production of Tissue Components

As discussed above, the disclosed tissue products can be produced using a variety of different synthetic or biologic materials that have been processed to have desired mechanical and biological properties.

The products can be formed from extracellular tissue matrix (ECM) based materials. For example, the ECM of tissue bone, skin, adipose tissue, dermis, intestine, urinary bladder, tendon, ligament, muscle, fascia, vascular, neurologic, vessel, liver, heart, lung, kidney, and cartilage tissue may be used to form part of the tissue products. A variety of methods are known in the art for obtaining tissue products (e.g., processed ECM materials), including dermal, small intestine-based, bone-based, and adipose tissue ECM. Suitable ECM materials are described, for example, in US Patent Publication No. US 20120310367 A1 to Connor et al. and US Patent Number 8,735, 054 to Sun et al.

An exemplary process for producing adipose tissue is described in US 2012/0310367A1. The method described therein can be used to produce adipose tissue-based products for incorporation with other materials or processed, as described above. The process generally includes obtaining adipose tissue ; mechanically processing the adipose tissue to produce small pieces; further processing the tissue to remove substantially all cellular material and/or lipids from the tissue; re-suspending the tissue in a solution to form a porous matrix or sponge; and cross-linking the tissue to produce a stable three-dimensional structure.

To assist in removal of the cellular components and produce a flowable mass, the tissue is first processed to produce small pieces. The material is cut, grinded, blended or otherwise mechanically treated to reduce the size of the tissue and/or to form a putty or flowable material. The adipose tissue can be treated using any repetitive cutting, grinding, or blending process. For example, in one embodiment, the tissue is first cut into relatively small pieces (e.g., about 2cm × 2cm). The pieces can them be placed in an aqueous suspension, which is treated with a blade grinder or similar instrument.

After processing, the tissue is then treated to removal cellular components and lipids. The cellular material can be removed by washing the material. For example, in some embodiments, the material is further diluted with water or another solvent. The diluted material is then centrifuged, and free lipids and cell debris will flow to the top, while the extracellular matrix proteins are deposited as a pellet. The protein pellet can then be re-suspended, and the washing and centrifugation can be repeated until a sufficient amount of the lipids and cellular materials are removed. In some cases, the process is repeated until substantially all cellular material and/or lipids are removed.

During, before, and/or after the washing steps, additional solutions or reagents can be used to process the material. For example, enzymes, detergents, and/or other agents may be used in one or more steps to remove cellular materials or lipids, remove antigenic materials, and/or reduce the bacteria or other bioburden of the material. For example, one or more washing steps can be included using detergents such as sodium dodecylsulfate or TRIS to assist in cell and lipid removal. In addition, enzymes such as lipases, DNAses, RNAses, alpha-galactosidase, or other enzymes can be used to ensure destruction of nuclear materials, antigens from xenogenic sources, and/or viruses. Further, peracetic acid solutions and/or peroxides can be used to help remove cellular materials and destroy bacteria or other potentially infectious agents.

After removal of cellular components, the material can then be formed into a porous or sponge-like material. Generally, the extracellular matrix is first re-suspended in an aqueous solvent. A sufficient amount of solvent is used to allow the material to form a liquid mass that can be poured. The amount of water added can be varied based on the desired porosity of the final material. In some cases, the re-suspended extracellular matrix may be mechanically treated by grinding, cutting, blending or other processes one or more additional times, and the treated material can be centrifuged and re-suspended one or more times to further remove cellular material or lipids (if needed) and/or to control the viscosity of the extracellular matrix suspension.

Once any additional washing and grinding steps are complete, the re-suspended material is placed in a container or mold to form the porous, sponge-like product. Generally, the porous or sponge-like material is formed by drying the material to leave a three-dimensional matrix with a porous structure. In some embodiments, the material is freeze-dried. Freeze-drying can allow production of a three-dimensional structure that generally conforms to the shape of the mold. In some cases, the materials can be re-suspended and the suspension can be mixed with other materials, as described above, and the mixtures can be dried to stabilize form the adipose component into a stable structure. For example, in one embodiment, the tissue product includes a polyurethane foam, and the adipose matrix suspension is poured into the foam and dried to form the final tissue product.

In some embodiments, the material is cross-linked. In some embodiments, the material is cross-linked after freeze drying. However, the material could also be cross-linked before or during the freeze-drying process. Cross-linking can be performed in a variety of ways. In one embodiment, cross-linking is accomplished by contacting the material with a cross-linking agent such as glutaraldehyde, genepin, carbodiimides, and diisocyantes. In addition, cross-linking can be performed by heating the material. For example, in some embodiments, the material can be heated to between 70ºC to 120ºC, or between 80ºC and 110ºC, or to about 100ºC, or any values between the specified ranges in a reduced pressure or vacuum. In addition, other cross-linking processes may be used to produce any of the disclosed products, including ultraviolet irradiation, gamma irradiation, and/or electron beam irradiation. In addition, a vacuum is not needed but may reduce cross-linking time. Further, lower or higher temperatures could be used as long as melting of the matrix proteins does not occur and/or sufficient time is provided for cross-linking.

### Use of Tissue Products

The tissue products described herein can be used to treat a variety of different anatomic sites. For example, as discussed throughout, the tissue products of the present disclosure are produced from composite materials that prevent compression, and thereby allow maintenance of implant volume after implantation. Accordingly, it is believed that the tissue products will provide superior regenerative capabilities when implanted in certain tissue sites, as compared to materials produced from other tissue types. In some cases, the tissue products can be implanted in tissue sites that are predominantly or significantly adipose tissue. In some cases, the tissue sites can include a breast (e.g., for augmentation, replacement of resected tissue, or placement around an implant).

In addition, any other site can be selected that will benefit from maintenance of tissue volume. For example, the tissue products may be used for reconstructive or cosmetic use in the face, buttocks, abdomen, hips, thighs, or any other site where maintenance of volume is desired. In any of those sites, the tissue may be used to reduce or eliminate wrinkles, sagging, or undesired shapes.

When used for breast tissue replacement or augmentation, the tissue can provide advantages over other tissue products. For example, the presently disclosed materials can be formed of a combination of components that permit adipose tissue ingrowth to support regeneration of naturally feeling breast tissue, e.g., after lumpectomy. In addition, the composite structure allows maintenance of implant tissue volume over time, while some other currently available materials suffer from excessive volume loss after implantation, thereby providing less than optimum aesthetic results.

### Example:

This study was performed to examine the in vivo (subcutaneous) response of three implants: (1) processed adipose tissue (AT), (2) polyurethane sponges (PU); and (3) PU embedded with AT (PAT).

AT was prepared as described in US Patent Publication 2012/0310367A1 (see Example at page 5). Human adipose tissue was obtained from cadaveric sources following all of the requirements of the American Association of Tissue Banks (AATB). Full thickness samples containing dermis and fat were obtained from AATB certified tissue banks. The adipose tissue was mechanically excised from the dermis and was placed in buffered saline and held at 4° C. until processing.

The adipose tissue, of various thicknesses, was cut into small pieces. The tissue was minced using a blade grinder, and the grinding process was repeated until all of the adipose pieces had been minced.

The minced adipose was suspended in water and centrifuged to separate the AT matrix from cellular debris and lipids. The centrifugation and pelleting were repeated until all materials were processed. All of the ECM pellets were combined, re-suspended in water, and washed by centrifugation three times.

The washed adipose ECM was re-suspended in water, and the suspension was washed using centrifugation as described above. The final ECM suspension was centrifuged to generate a final packed ECM pellet. The washed ECM pellet was slowly re-suspended in water. The slurry was dispersed into molds, freeze dried followed by DHT for 24 hours at 80 °C to crosslink and stabilize the AT.

PAT was prepared in the same manner as AT except that a commercially available 10 pore per inch polyurethane foam (PU) (UFP Technologies, 331 Patterson Avenue, Grand Rapids, MI 49512, Part Number 8467) was infiltrated with the AT slurry prior to freeze drying and DHT. The PU sponge was freeze dried and DHT processed to ensure it experienced the same conditions as PAT.

In this study, three athymic rats were implanted subcutaneously with one of each of the above-identified materials. The location of each implant type was varied among the rats to account for the possibility of implant location affecting implant results. The implants were removed at twenty-eight days, and evaluation consisted of explant weight measurements to approximate volume and gross and histological observations.

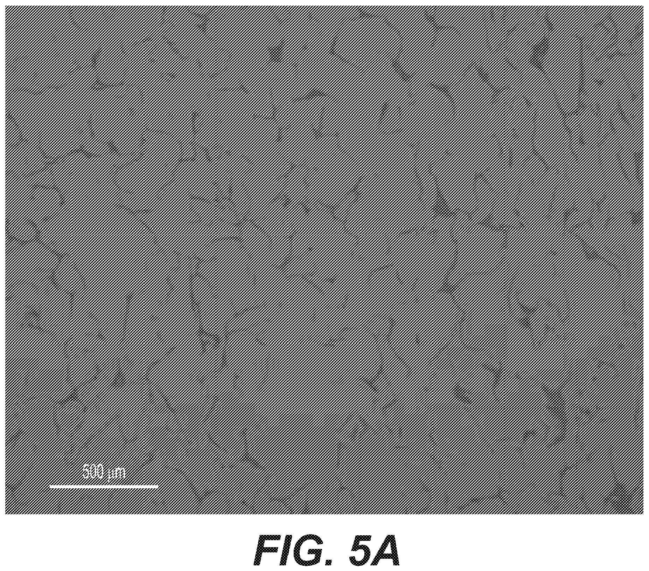

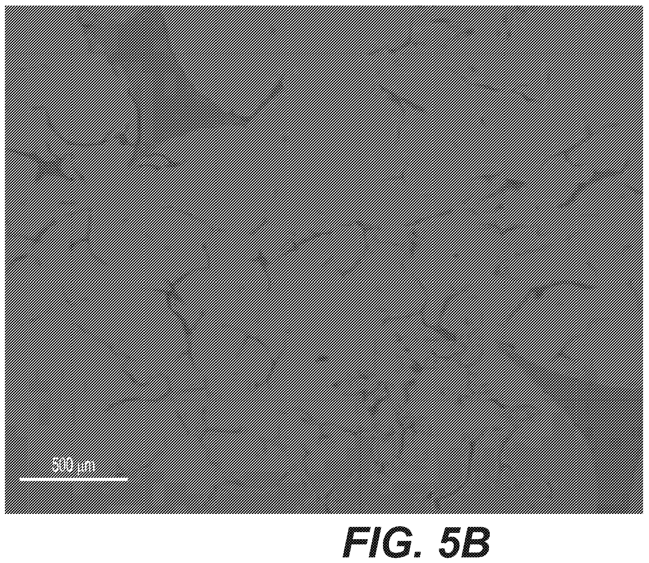

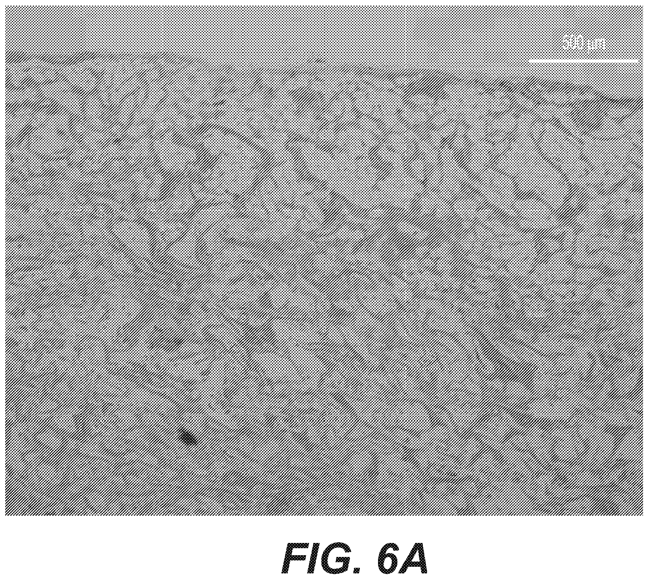

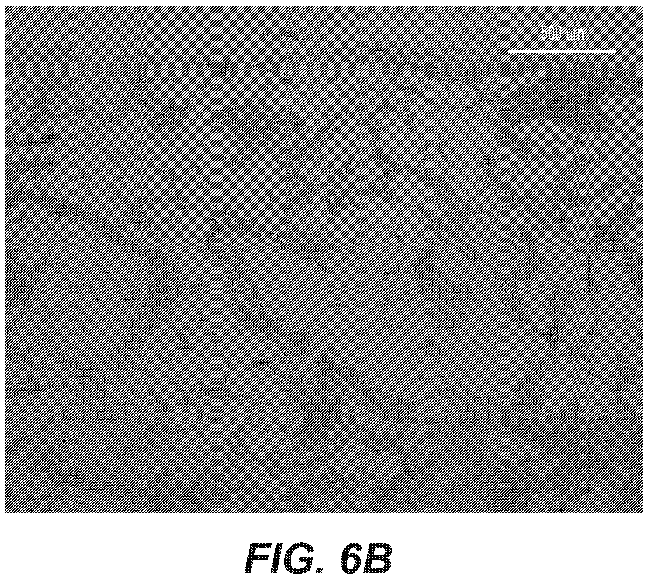

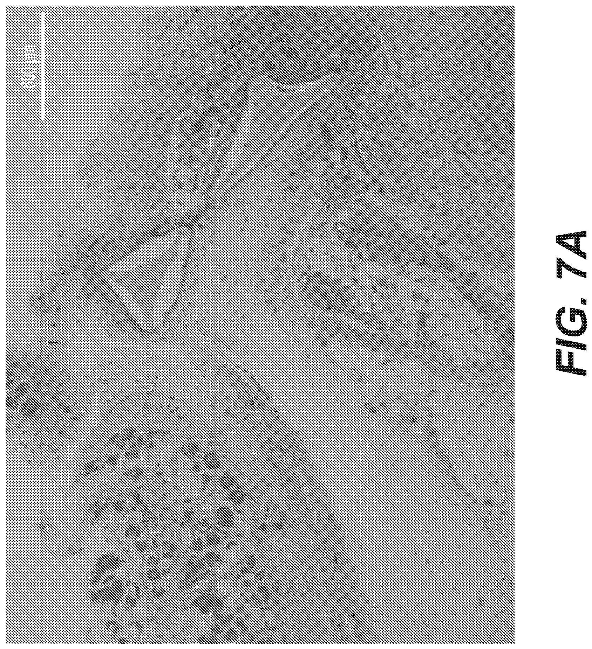

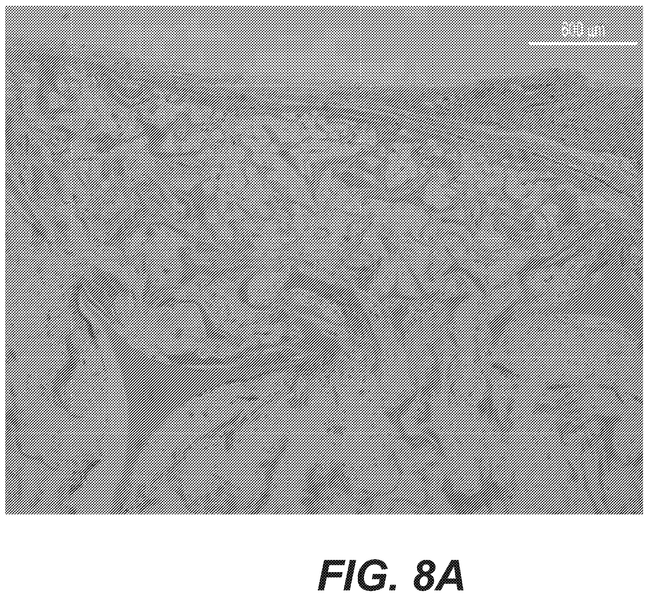

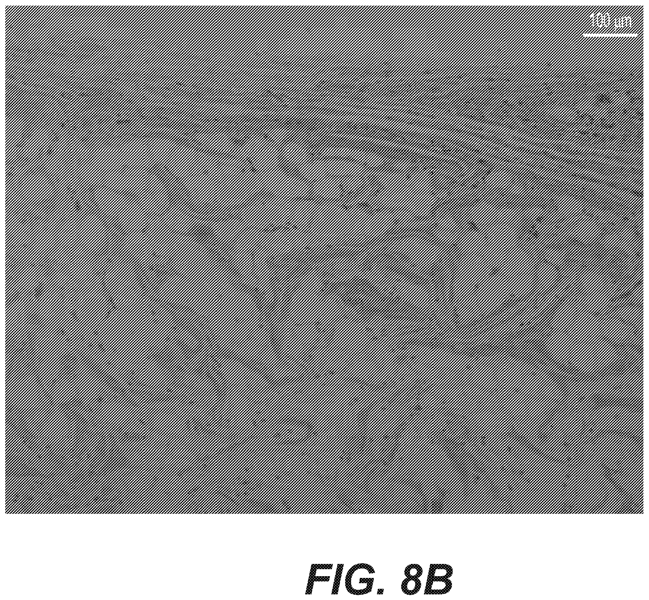

Fig. 5A is a hematoxylin and eosin (H&E) stained section of processed adipose tissue prior to implantation, as describe in the Example below. Fig. 5B is an H&E stained section of processed adipose tissue/polyurethane composite material prior to implantation, as describe in the Example below. Figs. 6A-6B are H&E stained sections of processed adipose tissue after explantation, as describe in the Example below. Figs 7A-7B are H&E stained sections of a polyurethane material after explantation, as describe in the Example below. Figs 8A-8B are H&E stained sections of processed adipose tissue/polyurethane composite material after explantation, as describe in the Example below.

The results showed that PAT maintained the majority of its volume, while AT lost approximately half of its volume. The histology showed that the AT within PAT retains its pre-implant structure, while the AT alone tends to become less open-indicating volume loss. The adipocyte deposition for AT by SME estimation ranged between 40 and greater than 80% for the three explants studies. The adipocyte deposition for PAT was estimated to be between 10 and 20% for the three explants. The lower deposition for PAT may be partially explained by the larger overall retained thickness and volume of the PAT which would require a further depth of infiltration. Finally the AT within PAT prevented infiltration of host connective tissue into the implant; this connective tissue infiltration was observed in the majority of the PU alone explants.

The PU and PAT groups appeared to be significantly larger than the AT group after explantation. The PU and PAT explants weights were approximately double the weights of the AT explants. The initial volume of the implants was approximately 1.15 cm³ since they had dimensions 0.8 x 0.8 x 1.2 cm. Since the density of the tissue with bodily fluids is approximately 1 g/cm³, the weight in grams approximates volume. The PAT and PU groups therefore maintained their volume compared to AT. The AT explants had the softest feel. which is expected based on AT being softer prior to implantation. The PU explants were considerably firmer than AT and also firmer than PAT.

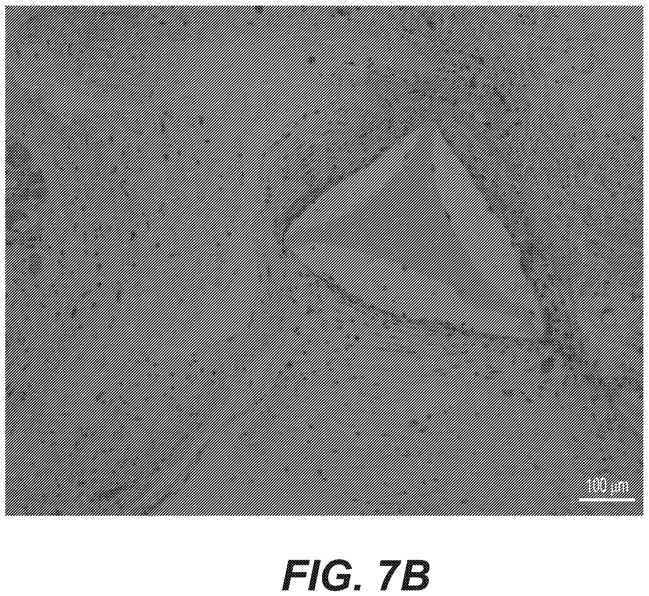

The AT group had the highest percentage of adipocyte deposition. The PU histology showed some muscle infiltration into the implant for all three explants; this muscle infiltration was not observed for PAT or AT. The PU also had host connective tissue infiltration throughout the explants which is expected for a macroporous open structure. There was adipocyte deposition into the edges of both PU and AT PU with some explants showing more than others. For example see Figure 7 which shows the PAT explants with the most robust adipocyte infiltration of the three PAT implants. The PAT group differed from PU in that the AT tissue appears to have blocked the infiltration of host connective tissue. The AT explants show collapse of the implant collagen structure in the areas where the adipose tissue has not infiltrated. The PAT collagen structure appears to be maintained.

Overall, the combination of PU and processed adipose tissue provides an advantage in that it permits adipose tissue ingrowth (like the AT produce), blocks connective tissue infiltration (unlike the PU), and maintains implant volume better that processed adipose tissue alone.

## Claims

1. A device for treatment of an anatomic defect, comprising:
a synthetic polymeric material substrate; and
an acellular tissue matrix embedded in the synthetic polymeric material substrate lattice framework, wherein the acellular tissue matrix comprises an adipose tissue matrix.

2. The device of claim 1, wherein the synthetic polymeric material substrate comprises polypropylene.

3. The device of claim 1, wherein the synthetic polymeric material substrate is selected from at least one of polyurethanes, degradable polyesters, polyglycolic acid, polylactic acid, polycaprolactone, polytrimethylene carbonate and copolymers, polyanhydrides, polycarbonates, polyesteramides, polyphosphazenese, polyolefins, nondegradable polyesters, nylon, polyacrylates, silicones, tyrosine base polymers, polyhydroxyalkanoates, chitin, or chitosan.

4. The device of any of claims 1-3, wherein the acellular tissue matrix is formed by a process comprising:
selecting an adipose tissue;
treating the tissue to remove substantially all cellular material from the tissue;
suspending the tissue in a liquid to form a suspension;
placing the synthetic polymeric material substrate in the suspension; and
freezing and drying the suspension to form a porous sponge embedded in a synthetic polymeric material substrate lattice framework.

5. The device of claim 4, wherein the process further comprises stabilizing the porous sponge.

6. The device of claim 5, wherein stabilizing the porous sponge comprises cross-linking the porous sponge and wherein cross-linking the porous sponge comprises performing at least one of chemical cross-linking, cross-linking with e-beam radiation, cross-linking with gamma radiation, or cross-linking with ultraviolet radiation.

7. The device of claim 5, wherein stabilizing the porous sponge comprises subjecting the porous sponge to a dehydrothermal process.

8. A method for producing a treatment device, comprising:
selecting an adipose tissue;
treating the tissue to remove substantially all cellular material from the tissue;
suspending the tissue in a liquid to form a suspension;
placing a synthetic polymeric material substrate in the suspension; and
freezing and drying the suspension to form a porous sponge embedded in a synthetic polymeric material substrate lattice framework.

9. The method of claim 8, wherein the synthetic polymeric material substrate comprises polypropylene.

10. The method of claim 8, wherein the synthetic polymeric material substrate is selected from at least one of polyurethanes, degradable polyesters, polyglycolic acid, polylactic acid, polycaprolactone, polytrimethylene carbonate and copolymers, polyanhydrides, polycarbonates, polyesteramides, polyphosphazenes, polyolefins, nondegradable polyesters, nylon, polyacrylates, silicones, tyrosine base polymers, polyhydroxyalkonoates, chitin, or chitosan.

11. The method of any of claims 8-10, wherein the method further comprises stabilizing the porous sponge.

12. The method of claim 11, wherein stabilizing the porous sponge comprises cross-linking the porous sponge and wherein cross-linking the porous sponge comprises performing at least one of chemical cross-linking, cross-linking with e-beam radiation, cross-linking the gamma radiation, or cross-linking with ultraviolet raditation.

13. The method of claim 11, wherein stabilizing the porous sponge comprises subjecting the porous sponge to a dehydrothermal process.

14. The method of any one of claims 8-14, wherein synthetic polymeric material substrate lattice framework is formed before the step of freezing and drying the suspension to form a porous sponge.

15. The method of any one of claims 8-14, wherein synthetic polymeric material substrate lattice framework is formed simultaneously with the step of freezing and drying the suspension to form a porous sponge.

## Patentansprüche

1. Vorrichtung für eine Behandlung eines anatomischen Defekts, die Folgendes umfasst:
ein Substrat aus synthetischem Polymermaterial; und
eine azelluläre Gewebematrix, die in das Gittergerüst des Substrats aus synthetischem Polymermaterial eingebettet ist, wobei die azelluläre Gewebematrix eine Fettgewebematrix umfasst.

2. Vorrichtung nach Anspruch 1, wobei das Substrat aus synthetischem Polymermaterial Polypropylen umfasst.

3. Vorrichtung nach Anspruch 1, wobei das Substrat aus synthetischem Polymermaterial aus Polyurethanen, abbaubaren Polyestern, Polyglykolsäure, Polymilchsäure, Polycaprolacton, Polytrimethylencarbonat und Copolymeren, Polyanhydriden, Polycarbonaten, Polyesteramiden, Polyphosphazenen, Polyolefinen, nicht abbaubaren Polyestern, Nylon, Polyacrylaten, Silikonen, Polymeren auf Tyrosinbasis, Polyhydroxyalkanoaten, Chitin und/oder Chitosan ausgewählt ist.

4. Vorrichtung nach einem der Ansprüche 1-3, wobei die azelluläre Gewebematrix durch einen Vorgang ausgebildet wird, der Folgendes umfasst:
Auswählen eines Fettgewebes;
Behandeln des Gewebes, um im Wesentlichen das gesamte Zellmaterial aus dem Gewebe zu entfernen;
Suspendieren des Gewebes in einer Flüssigkeit, um eine Suspension auszubilden;
Platzieren des Substrats aus synthetischem Polymermaterial in der Suspension; und
Einfrieren und Trocknen der Suspension, um einen porösen Schwamm auszubilden, der in ein Gittergerüst des Substrats aus synthetischem Polymermaterial eingebettet ist.

5. Vorrichtung nach Anspruch 4, wobei der Vorgang ferner ein Stabilisieren des porösen Schwamms umfasst.

6. Vorrichtung nach Anspruch 5, wobei das Stabilisieren des porösen Schwamms ein Vernetzen des porösen Schwamms umfasst und wobei das Vernetzen des porösen Schwamms ein Durchführen eines chemischen Vernetzens, Vernetzens mit Elektronenstrahlstrahlung, Vernetzens mit Gammastrahlung und/oder Vernetzens mit ultravioletter Strahlung umfasst.

7. Vorrichtung nach Anspruch 5, wobei das Stabilisieren des porösen Schwamms umfasst, den porösen Schwamm einem dehydrothermischen Vorgang zu unterziehen.

8. Verfahren zum Herstellen einer Behandlungsvorrichtung, das Folgendes umfasst:
Auswählen eines Fettgewebes;
Behandeln des Gewebes, um im Wesentlichen das gesamte Zellmaterial aus dem Gewebe zu entfernen;
Suspendieren des Gewebes in einer Flüssigkeit, um eine Suspension auszubilden;
Platzieren eines Substrats aus synthetischem Polymermaterial in der Suspension; und
Einfrieren und Trocknen der Suspension, um einen porösen Schwamm auszubilden, der in ein Gittergerüst des Substrats aus synthetischem Polymermaterial eingebettet ist.

9. Verfahren nach Anspruch 8, wobei das Substrat aus synthetischem Polymermaterial Polypropylen umfasst.

10. Verfahren nach Anspruch 8, wobei das Substrat aus synthetischem Polymermaterial aus Polyurethanen, abbaubaren Polyestern, Polyglykolsäure, Polymilchsäure, Polycaprolacton, Polytrimethylencarbonat und Copolymeren, Polyanhydriden, Polycarbonaten, Polyesteramiden, Polyphosphazenen, Polyolefinen, nicht abbaubaren Polyestern, Nylon, Polyacrylaten, Silikonen, Polymeren auf Tyrosinbasis, Polyhydroxyalkanoaten, Chitin und/oder Chitosan ausgewählt ist.

11. Verfahren nach einem der Ansprüche 8-10, wobei das Verfahren ferner das Stabilisieren des porösen Schwamms umfasst.

12. Verfahren nach Anspruch 11, wobei das Stabilisieren des porösen Schwamms das Vernetzen des porösen Schwamms umfasst und wobei das Vernetzen des porösen Schwamms das Durchführen eines chemischen Vernetzens, Vernetzens mit Elektronenstrahlstrahlung, Vernetzens der Gammastrahlung und/oder Vernetzens mit ultravioletter Strahlung umfasst.

13. Verfahren nach Anspruch 11, wobei das Stabilisieren des porösen Schwamms umfasst, den porösen Schwamm einem dehydrothermischen Vorgang zu unterziehen.

14. Verfahren nach einem der Ansprüche 8-14, wobei ein Gittergerüst des Substrats aus synthetischem Polymermaterial vor dem Schritt des Einfrierens und Trocknens der Suspension ausgebildet wird, um einen porösen Schwamm auszubilden.

15. Verfahren nach einem der Ansprüche 8-14, wobei das Gittergerüst des Substrats aus synthetischem Polymermaterial gleichzeitig mit dem Schritt des Einfrierens und Trocknens der Suspension ausgebildet wird, um einen porösen Schwamm auszubilden.

## Revendications

1. Dispositif de traitement d'un défaut anatomique, comprenant :
un substrat en matériau polymère synthétique ; et
une matrice de tissu acellulaire incorporée dans la structure en treillis de substrat en matériau polymère synthétique, la matrice de tissu acellulaire comprenant une matrice de tissu adipeux.

2. Dispositif selon la revendication 1, dans lequel le substrat en matériau polymère synthétique comprend du polypropylène.

3. Dispositif selon la revendication 1, dans lequel le substrat en matériau polymère synthétique est choisi parmi des polyuréthanes, et/ou des polyesters dégradables, et/ou de l'acide polyglycolique, et/ou de l'acide polylactique, et/ou du polycaprolactone, et/ou du carbonate et des copolymères de polytriméthylène, et/ou des polyanhydrides, et/ou des polycarbonates, et/ou des polyesteramides, et/ou du polyphosphazène, et/ou des polyoléfines, et/ou des polyesters non dégradables, et/ou du nylon, et/ou des polyacrylates, et/ou des silicones, et/ou des polymères à base de tyrosine, et/ou des polyhydroxyalcanoates, et/ou de la chitine et/ou du chitosane.

4. Dispositif selon l'une quelconque des revendications 1 à 3, dans lequel la matrice de tissu acellulaire est formée par un procédé comprenant :
la sélection d'un tissu adipeux ;
le traitement du tissu pour éliminer sensiblement tout le matériau cellulaire du tissu ;
la suspension du tissu dans un liquide pour former une suspension ;
le placement du substrat en matériau polymère synthétique dans la suspension ; et
la congélation et le séchage de la suspension pour former une éponge poreuse incorporée dans une structure en treillis de substrat en matériau polymère synthétique.

5. Dispositif selon la revendication 4, dans lequel le procédé comprend en outre la stabilisation de l'éponge poreuse.

6. Dispositif selon la revendication 5, dans lequel la stabilisation de l'éponge poreuse comprend la réticulation de l'éponge poreuse et dans lequel la réticulation de l'éponge poreuse comprend l'exécution d'une réticulation chimique, et/ou d'une réticulation par irradiation par faisceau d'électrons, et/ou d'une réticulation par rayonnement gamma, et/ou d'une réticulation par rayonnement ultraviolet.

7. Dispositif selon la revendication 5, dans lequel la stabilisation de l'éponge poreuse comprend la soumission de l'éponge poreuse à un processus déshydrothermique.

8. Procédé de production d'un dispositif de traitement, comprenant :
la sélection d'un tissu adipeux ;
le traitement du tissu pour éliminer sensiblement tout le matériau cellulaire du tissu ;
la suspension du tissu dans un liquide pour former une suspension ;
le placement d'un substrat en matériau polymère synthétique dans la suspension ; et
la congélation et le séchage de la suspension pour former une éponge poreuse incorporée dans une structure en treillis de substrat en matériau polymère synthétique.

9. Procédé selon la revendication 8, dans lequel le substrat en matériau polymère synthétique comprend du polypropylène.

10. Procédé selon la revendication 8, dans lequel le substrat en matériau polymère synthétique est choisi parmi des polyuréthanes, et/ou des polyesters dégradables, et/ou de l'acide polyglycolique, et/ou de l'acide polylactique, et/ou du polycaprolactone, et/ou du carbonate et des copolymères de polytriméthylène, et/ou des polyanhydrides, et/ou des polycarbonates, et/ou des polyesteramides, et/ou du polyphosphazène, et/ou des polyoléfines, et/ou des polyesters non dégradables, et/ou du nylon, et/ou des polyacrylates, et/ou des silicones, et/ou des polymères à base de tyrosine, et/ou des polyhydroxyalcanoates, et/ou de la chitine et/ou du chitosane.

11. Procédé selon l'une quelconque des revendications 8 à 10, dans lequel le procédé comprend en outre la stabilisation de l'éponge poreuse.

12. Procédé selon la revendication 11, dans lequel la stabilisation de l'éponge poreuse comprend la réticulation de l'éponge poreuse et dans lequel la réticulation de l'éponge poreuse comprend l'exécution d'une réticulation chimique, et/ou d'une réticulation par irradiation par faisceau d'électrons, et/ou d'une réticulation par rayonnement gamma, et/ou d'une réticulation par rayonnement ultraviolet.

13. Procédé selon la revendication 11, dans lequel la stabilisation de l'éponge poreuse comprend la soumission de l'éponge poreuse à un processus déshydrothermique.

14. Procédé selon l'une quelconque des revendications 8 à 14, dans lequel la structure en treillis de substrat en matériau polymère synthétique est formée avant l'étape de congélation et de séchage de la suspension pour former une éponge poreuse.

15. Procédé selon l'une quelconque des revendications 8 à 14, dans lequel la structure en treillis de substrat en matériau polymère synthétique est formée simultanément avec l'étape de congélation et de séchage de la suspension pour former une éponge poreuse.
